# EUROPEAN PATENT APPLICATION

(11) **EP 2 189 154 A1**
(43) Date of publication of application: **26.05.2010**
(21) Application number: 08169848.2
(22) Date of filing: 25.11.2008
(51) Int. Cl.: A61K 9/16, A61K 9/20, A61K 9/26, A61K 31/7076, A61K 47/02, A61K 47/12

(54) **Effervescent tablets and mouth-soluble granulates of S-adenosyl methionine and process for the preparation thereof**

(71) Applicant: Gnosis S.p.A., 20121 Milano (IT)
(72) Inventor: Bianchi, Davide, 20033, DESIO (MI) (IT); Berna, Marco, 20033, DESIO (MI) (IT)
(74) Representative: Minoja, Fabrizio

(57) **Abstract**

A pharmaceutical or nutraceutical composition in form of effervescent tablets or mouth-soluble granulates comprising SAMe, an acid, a carbon dioxide source, calcium oxide and optionally pharmaceutically acceptable excipients.

## Description

The present invention refers to effervescent tablets and mouth-soluble granulates of S-adenosyl methionine.

### Background of the invention

S-Adenosyl methionine (SAMe) is a physiological donor of methyl groups present in all living organisms, involved in enzyme transmethylation reactions.

This substance is essentially used in clinical practice as an antidepressant.

"SAMe" refers both to the racemic mixture and to the individual diastereoisomers (RS)-(+)-S-adenosyl-L-methionine [(RS)-(+)-SAMe)] and (SS)-(+)-S-adenosyl-L-methionine [(SS)-(+)-SAMe)], as well as to mixtures other than the racemic mixture.

The use of S-adenosyl methionine as a drug and/or dietetic is problematic because it is extremely unstable at temperatures above 0°C or in the presence of moisture, resulting either in the degradation of the active ingredient, considered as the sum of the two diastereoisomers, or in the conversion of active (SS)-(+)-S-adenosyl-L-methionine to the inactive (RS)-(+) isomer.

Italian Patent 829906 describes a process for the preparation of pharmaceutically acceptable salts of (SS,RS)-S-adenosyl-L-methionine with quantities of the inactive (RS)-(+) diastereoisomer lower than 3%. It is also important to use racemic mixtures with a high percentage of the S,S diastereoisomer which is the only one pharmacologically active. However, the patent confirms that although more than 97% of active S,S diastereoisomer is obtained at ambient temperature, the racemic mixture is unstable over time, with conversion of the (SS)-(+) isomer into the (RS) isomer in a relatively short time.

US 354363, US 136271 and US 354263 disclose a method for stabilising pharmaceutically acceptable salts of S-adenosyl methionine comprising S-adenosyl methionine para-toluene sulphonate, S-adenosyl methionine-1,4-butene disulphonate, S-adenosyl methionine sulphate, S-adenosyl methionine tosylate with a group of substances comprising chitosan, dextran, carboxymethylcellulose, fumaric acid, azelaic acid and tryptophan. In particular the first of these patents indicates that it is important to have a product with the highest possible amount of S,S diastereoisomer, the R,S diastereoisomer being not only inactive but even opposing the pharmacological effect of the S,S diastereoisomer.

US 136271 and US 354363 disclose methods for stabilising S-adenosyl methionine salts using the abovementioned substances in a percentage by weight with respect to the active ingredient which is much higher than 50%, and adding them in reconstituted aqueous solution to S-adenosyl methionine salts, with final lyophilisation. This involves high production costs and very low yields because the % of ions in the final product falls from approximately 50% to approximately 25%.

US 3954726 and US 4057672 disclose comparatively stable salts of S-adenosyl methionine, up to 25°C and 45°C, respectively. US 4465672 describes stable salts of S-adenosyl methionine with 5 mols of a sulphonic acid with a pK of less than 2.5, obtained by a process comprising the preparation of a concentrated aqueous solution of an impure salt of SAMe, purification of the solution and its elution with a dilute aqueous solution of the preselected sulphonic acid, titration of the resulting eluate, concentration and lyophilisation or spray-drying. Due to the high instability of SAMe, the use of an aqueous environment is an obvious disadvantage, and even if residual moisture content is successfully contained it is still unacceptable.

WO2007/113885 discloses a method for stabilising a solid oral composition of SAMe and/or NADH or their salts, by using calcium oxide or hydroxide optionally in combination with malic acid, glutamic acid, xylitol, calcium sulphate hemihydrate, magnesium oxide and/or mixtures thereof.

Effervescent tablets and mouth-soluble granulates are convenient, attractive, easy to use premeasured dosage forms. These advantages, however, also involve some technological problems, the two most important of which are hygroscopicity and lubrication.

The instability of effervescent tablets and mouth-soluble granulates, their tendency to absorb moisture and lose reactivity are generally known. Due to this instability in the presence of water, conventional wet granulation and the subsequent granulate compression are hardly applicable.

Sometimes the granulation has been carried out using very low amounts of water, for example by melting the citric acid monohydrate, which upon heating releases part of the crystallisation water which acts as the granulating fluid. Thus the granulate must be processed in conditions of severely controlled relative humidity, usually lower than 20%.

Alternatively, techniques of dry granulation, in the absence of aqueous phases, have been applied using volatile organic solvents like ethanol. However, such techniques require special manufacturing environments with strictly controlled conditions of relative humidity (normally lower than 20%) and with explosion-proof equipment.

Another technique, which is more time-consuming and more laborious, is represented by the separated wet granulation of acidic and alkali granules, which are subsequently mixed and compressed to give the final composition.

The direct compression of the simple physical blend of the components of the formulation was proposed to obviate the above difficulties. However such an operation has to be carried out in controlled thermo-hygrometric conditions, for example at temperatures lower than 20-25°C and with relative humidity lower than 20%, using tabletting machines with tapered dies and punches coated with chromium alloys.

Other important technological problems affecting the manufacture of effervescent tablets and mouth-soluble granulates concern lubrication, since the lubricant must not only have lipophilic properties for good lubrication, but also high water solubility, to give adequate disintegration and quickly produce a clear solution. Most substances used as lubricants, such as magnesium stearate, although effective, are water insoluble. The resulting solution after disintegration is cloudy and has often a soapy taste. Ideally, non toxic lubricants with high water solubility and acceptable taste are required. Moreover, the effervescent base is inherently difficult to lubricate, partly due to the nature of the raw materials used and partly due to the rapid tablet disintegration usually required which limits the use of high percentages of lubricants.

In view of the above mentioned technical problems, it will be appreciated that previous teachings related to non-effervescent oral solid dosage forms are not applicable to effervescent formulations.

### Description of the invention

It has now surprisingly been found that the stable effervescent tablets and mouth-soluble granulates of S-adenosyl methionine (SAMe) may be obtained by mixing calcium oxide with a carbon dioxide source (usually a carbonate or bicarbonate), an acid and optionally other excipients.

The effervescent tablets and mouth-soluble granulates of the invention can be prepared by direct compression in normal thermo-hygrometric conditions and with standard tabletting equipment.

It has also been found that this technology may be applied also to active ingredients and/or excipients which cannot be wet-granulated or which contain a residual percentage of hardly eliminable crystallisation water.

In addition, the use of calcium oxide is particularly advantageous for the preparation of effervescent tablets and mouth-soluble granulates containing a cyclodextrin as the component of an inclusion complex or as an excipient, notwithstanding the hygroscopic properties of cyclodextrins.

SAMe is preferably S-adenosyl methionine para-toluene sulphonate, S-adenosyl methionine-1,4-butene disulphonate, S-adenosyl methionine sulphate, S-adenosyl methionine tosylate, S-adenosyl methionine phytate.

The acidic component in the formulations of the invention is selected from citric, tartaric, malic, maleic, fumaric or adipic acid. Anhydrous citric acid and fumaric acid are preferred, in the form of a salt such as mono sodium or potassium fumarate. Fumaric acid allows to decrease the quantity of lubricant.

Another preferred acid is maleic acid optionally present as a salt. In some cases mixtures of acids and/or salts are particularly suitable to modulate either the strength of the acid or the lubricant properties.

The carbon dioxide source is selected from an alkali or alkaline-earth metal carbonate or bicarbonate or sodium glycine carbonate or mixtures thereof. Examples of suitable carbon dioxide sources include sodium carbonate, potassium carbonate, sodium bicarbonate, potassium bicarbonate, calcium carbonate, sodium glycine carbonate.

Calcium oxide is mixed with said carbon dioxide sources, particularly carbonates and/or bicarbonates.

The compositions of the invention may optionally comprise at least one further active ingredient, preferably selected from phosphatidylserine and/or DL-phosphorylserine and/or N-acetyl-L-glutamine and/or L - Carnitine and/or their mixtures.

The formulation may comprise other excipients such as:
a lubricant selected from sodium benzoate, sodium and potassium fumarate, leucine, alanine, PEG 200 - 8000;
a sweetening agent selected from sucralose, aspartame, saccharin, cyclamate, sugars, preferably sucralose;
a diluent selected from lactose, mannitol, xylitol, sorbitol or mixtures thereof and preferably granular xylitol and optionally flavouring agents, ligands, preservatives or others.

Granulated xylitol is particularly preferred as a diluent since it facilitates the blend flowability thereby improving compressibility and machinability.

Xylitol, sorbitol, lactose, mannitol, sugar, dextrose, leucine, alanine or mixtures thereof are particularly preferred. Leucine and/or alanine and/or PEG 200 - 8000 are particularly preferred.

The compositions of the invention contain SAMe or its salts in a quantity from 10 to 95% by weight, preferably from 30 to 90% by weight of the composition.

The mixture of calcium oxide/bicarbonate and/or carbonate is generally present in a quantity from 1 to 50% by weight, preferably between 2 and 40% by weight of the composition.

The effervescent blend of the invention allows the preparation of highly soluble, stable and small-sized effervescent granulates and mouth-soluble tablets, by direct compressing the components mixture at the standard thermo-hygrometric conditions of normal pharmaceutical production facilities, using standard tabletting machines with normal punches and dies. Also the subsequent processing, storage and packaging can be performed at normal temperature and moisture conditions.

The effervescent compositions of the invention solubilize on contact with water and produce a clear solution for oral administration.

The possibility to prepare a tablet by direct compression and to obtain a rapid disintegration represents a remarkable formulation improvement.

Other advantages of the composition are the low content of sodium ions, and a less fizzy effervescence, more pleasant to the patient.

Moreover the composition of the invention, thanks to its small size, mild effervescence and rapid disintegration, can also be prepared as fast dissolving tablet. In fact, when introduced in the mouth, the tablet comes in contact with saliva, disintegrates and rapidly forms an easily swallowable solution or aqueous dispersion.

The compositions according to this invention may be in the form of tablets, capsules, granules and/or powders. Preferably, the compositions of the invention are in the form of effervescent tablets and mouth-soluble granulates.

A further object of this invention is a process for the preparation of effervescent tablets and mouth-soluble granulates comprising SAMe, or their salts, which comprises the following steps:
a) mixing of the SAMe, or its salts, with a mixture of calcium oxide/bicarbonate and/or carbonate and pharmaceutically acceptable excipients;
b) precompression and subsequent granulation of the mixture from a);
c) mixing of the granulate from b) with an acid selected from malic acid, adipic acid, citric acid, fumaric acid and optionally with pharmaceutically acceptable excipients such as xylitol, sorbitol, lactose, mannitol, sugar, dextrose, leucine, alanine, PEG 200 - 8000 or mixtures thereof;
d) compression of the mixture from c), with the optional addition of sweeteners and/or flavourings.

The process is carried out in an environment in which the relative humidity is kept below 20% and the temperature is from 18 to 25°C, preferably about 20°C.

Granulation is preferably carried out using a rotating blade granulator fitted with a stainless mesh having holes from 1.2 mm to 3.2 mm in diameter.

More particularly, in step a) the active ingredient is preferably mixed with a mixture of calcium oxide/bicarbonate and/or carbonate from approximately 1.0 to approximately 30% by weight and/or fumaric acid from approximately 0.5 to approximately 10% by weight and/or PEG 200 - 8000 from approximately 0.5 to approximately 10% by weight and/or glycine from approximately 0.5 to approximately 2.0% by weight with respect to the active ingredient.

In step c), the granulate from b) is preferably mixed with malic acid from approximately 1.0 to 10.0% by weight and/or adipic acid from approximately 1.0 to approximately 20.0% by weight and/or leucine from approximately 1.0 to approximately 10% by weight and/or alanine from approximately 1 to approximately 10% by weight of the active ingredient.

The optional further active ingredient such as phosphatidylserine, DL-phosphorylserine and/or N-acetyl-L-glutamine and/or L - Carnitine may be added in step c).

The invention is further illustrated in the following examples.

### EXAMPLE 1

Effervescent tablets of 200 mg SAMe ion/tablet

Composition based on SAMe sulphate p-toluene sulphonate

| | |
|---|---|
| A. SAMe sulphate p-toluene sulphonate | 400.00 mg |
| B. Calcium oxide | 20.00 mg |
| C. Anhydrous Calcium chloride | 20.00 mg |
| D. Sodium carbonate | 75.00 mg |
| E. Anhydrous Citric acid | 40.00 mg |
| F. Anhydrous Malic acid | 30.00 mg |
| G. Sorbitol | 270.00 mg |
| H. Mannitol | 119.00 mg |
| I. Sucralose | 1.00 mg |
| L. Lemon Flavor | 3.00 ng |
| M. Alanine | 22.00 mg |
| Total weight of effervescent tablet | 1000.00 mg |

### EXAMPLE 1A

Effervescent tablets of 200 mg SAMe ion/tablet

Composition based on SAMe sulphate p-toluene sulphonate

| | |
|---|---|
| A. SAMe sulphate p-toluene sulphonate | 400.00 mg |
| B. Calcium oxide not present | 00.00 mg |
| C. Anhydrous Calcium chloride | 20.00 mg |
| D. Sodium carbonate | 75.00 mg |
| E. Anhydrous Citric acid | 40.00 mg |
| F. Anhydrous Malic acid | 30.00 mg |
| G. Sorbitol | 290.00 mg |
| H. Mannitol | 119.00 mg |
| I. Sucralose | 1.00 mg |
| L. Lemon Flavor | 3.00 ng |
| M. Alanine | 22.00 mg |
| Total weight of effervescent tablet | 1000.00 mg |

### 1.1. Mixing A

The working environment was conditioned to a temperature of 20°C and a relative humidity value of approximately 20% RH. A, B and C were then transferred to the mixer in the quantities indicated above, leaving under stirring for approximately 30 minutes. At the end of this operation the mixture was transferred to dry containers, always controlling moisture content and temperature.

### 1.2. Mixing B

The working environment was conditioned to a temperature of 20°C and a relative humidity value of approximately 20% RH. D, E, F, G, H, I, and L were then transferred to the mixer in the quantities indicated above, leaving under stirring for approximately 30 minutes. At the end of this operation the mixture was transferred to dry containers, always controlling moisture content and temperature.

### 1.3. Mixing C

The working environment was conditioned to a temperature of 20°C and a relative humidity value of approximately 20% RH. Mixing A, Mixing B and 50% of M were then transferred to the mixer in the quantities indicated above, leaving under stirring for approximately 30 minutes. At the end of this operation the resulting mixture was transferred to dry containers, always controlling moisture content and temperature.

### 1.4. Precompression

Precompression of the mixture C was effected using a rotary machine equipped with round punches of 25.0 mm. The hardness of the tablets had to be regulated to subsequently produce a granulate having good flow characteristics.

### 1.5 Granulation

The tablets produced during the first processing stage were granulated on a 1000-1500 µm mesh, again in a humidity-controlled environment.

### 1.6 Mixing

The granulate obtained in stage 1.5 was transferred into the mixer, adding 50% of fumaric acid and leaving under stirring for approximately 30 minutes. At the end of this operation the resulting mixture was transferred into dry containers.

### 1.7 Compression

Final compression of the granulate was carried out using a rotary machine equipped with round punches of 16 mm adjusting the weight to 1100 mg/tablet and the compression force to at least 25 KP. The tablets had a hardness of between 25 and 35 Kp.

Friability: ≤ 1.0%; disaggregation time: ≤ 3 minutes (measured using the method described in U.S.P. 24^{th} ed.).

Moisture content according to K.F. ≤ 1.00%.

Stability tests on effervescent tablets were performed at only 60°C for one month and for a single batch. The samples were stored in alu/alu blister

**Table 1**

| Batch 001 - effervescent tablets of 200 mg SAMe ion/tablet (qualitative/quantitative composition in Example 1) | | | | | |
|---|---|---|---|---|---|
| Batch (T/t)¹ | Moisture content % (K. Fischer) | S,S % | AD² (%) | MTAD³ (%) | SAMe⁴ |
| 001 (20/0) | 0.54 | 82.2 | 0.32 | 0.45 | 202.23 |
| 001A (60/1) | 0.43 | 66.32 | 2.21 | 4.34 | 199.43 |

¹ Temperature (°C)/time (months); ² adenosine; ³ methylthioadenosine; ⁴ SAMe sulphate p-toluene sulphonate (mg/tablet);

The data in Table 1 show that the tablets have optimum stability.

**Table 2**

| Batch 001A - effervescent tablets of 200 mg SAMe ion/tablet (qualitative/quantitative composition in Example 1A) | | | | | |
|---|---|---|---|---|---|
| Batch (T/t)¹ | Moisture content % (K. Fischer) | S,S % | AD² (%) | MTAD³ (%) | SAMe⁴ |
| 001A (20/0) | 0.76 | 81.7 | 0.32 | 0.76 | 202.23 |
| 001AA (60/1) | 0.87 | 50.65 | 14.56 | 16.94 | 136.93 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

The data in Table 2 show that the tablets are not stable.

### EXAMPLE 2

Mouth-soluble granulates of 200 mg SAMe ion/bags

Composition based on SAMe sulphate p-toluene sulphonate

| | |
|---|---|
| A. SAMe sulphate p-toluene sulphonate | 401.10 mg |
| B. Calcium oxide | 19.80 mg |
| C. Anhydrous Calcium chloride | 19.80 mg |
| D. Calcium carbonate | 74.89 mg |
| E. Anhydrous Citric acid | 37.40 mg |
| F. Anhydrous Malic acid | 37.40 mg |
| G. Xylitol 200 | 264.77 mg |
| H. Anhydrous granular Sorbitol | 220.00 mg |
| I. Sucralose | 0.88 mg |
| L. Lemon Flavor | 2.75 ng |
| M. Furaric acid | 22.00 mg |
| Total weight of bags | 1100.00 mg |

### EXAMPLE 2A

Mouth-soluble granulates of 200 mg SAMe ion/bags

Composition based on SAMe sulphate p-toluene sulphonate

| | |
|---|---|
| A. SAMe sulphate p-toluene sulphonate | 401.10 mg |
| B. Calcium oxide (not present) | 00.00 mg |
| C. Anhydrous Calcium chloride | 19.80 mg |
| D. Anhydrous Calcium carbonate | 74.89 mg |
| E. Anhydrous Citric acid | 37.40 mg |
| F. Anhydrous Malic acid | 37.40 mg |
| G. Xylitol 200 | 264.77 mg |
| H. Anhydrous granular Sorbitol | 239.80 mg |
| I. Sucralose | 0.88 mg |
| L. Lemon Flavor | 2.75 ng |
| M. Fumaric acid | 22.00 mg |
| Total weight of bags | 1100.00 mg |

### 1.1. Mixing A

The working environment was conditioned to a temperature of 20°C and a relative humidity value of approximately 20% RH. A, B and C were then transferred to the mixer in the quantities indicated above, leaving under stirring for approximately 30 minutes. At the end of this operation the mixture was transferred to dry containers, always controlling moisture content and temperature.

### 1.2. Mixing B

The working environment was conditioned to a temperature of 20°C and a relative humidity value of approximately 20% RH. D, E, F, G, H, I, and L were then transferred to the mixer in the quantities indicated above, leaving under stirring for approximately 30 minutes. At the end of this operation the resulting mixture was transferred to dry containers, always controlling moisture content and temperature.

### 1.3. Mixing C

The working environment was conditioned to a temperature of 20°C and a relative humidity value of approximately 20% RH. Mixing A, Mixing B and 100% of M were then transferred to the mixer in the quantities indicated above, leaving them under stirring for approximately 30 minutes. At the end of this operation the mixture was transferred to dry containers, always controlling moisture content and temperature.

### 1.4. Precompression

Precompression of the mixture C was effected using a rotary machine equipped with round punches of 25.0 mm. The hardness of the tablets produced had to be regulated to subsequently produce a granulate having good flow characteristics.

### 1.5 Granulation

The tablets produced during the first processing stage were granulated on a 1000-1500 µm mesh, again in a humidity-controlled environment.

Dissolution time: ≤ minutes (measured using the method described in U.S.P. 28^{th} ed.)

Moisture content according to K.F. ≤ 1.00%

Stability tests on effervescent tablets were performed at only 60°C for one months and for a single batch. The samples were stored in alu/alu bags.

**Table 3**

| Batch 002 - mouth-soluble granulates of 200 mg SAMe ion/bags (qualitative/quantitative composition in Example 2) | | | | | |
|---|---|---|---|---|---|
| Batch (T/t)¹ | Moisture content % (K. Fischer) | S,S % | AD² (%) | MTAD³ (%) | SAMe⁴ |
| 002 (20/0) | 0.57 | 80.1 | 0.22 | 0.32 | 204.54 |
| 002A (60/1) | 0.49 | 65.52 | 2.51 | 5.33 | 198.76 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

The data in Table 2 show that the tablets have optimum stability.

**Table 4**

| Batch 002A - mouth-soluble granulates of 200 mg SAMe ion/bags (qualitative/quantitative composition in Example 2A) | | | | | |
|---|---|---|---|---|---|
| Batch (T/t)¹ | Moisture content % (K. Fischer) | S,S % | AD² (%) | MTAD³ (%) | SAMe⁴ |
| 002A (20/0) | 0.55 | 82.4 | 0.29 | 0.43 | 203.74 |
| 002AA (60/1) | 0.86 | 51.72 | 12.76 | 18.09 | 123.98 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

The data in Table 2 show that the tablets are not stable.

### EXAMPLE 3

Effervescent tablets of 400 mg SAMe ion/tablet

Composition based on SAMe sulphate p-toluene sulphonate

| | |
|---|---|
| A. SAMe sulphate p-toluene sulphonate | 800.00 mg |
| B. Calcium oxide | 40.00 mg |
| C. Anhydrous Calcium chloride | 40.00 mg |
| D. Sodium bicarbonate | 70.00 mg |
| E. Anhydrous Tartaric acid | 45.00 mg |
| F. Anhydrous Malic acid | 35.00 mg |
| G. Sorbitol | 265.00 mg |
| H. Mannitol | 119.00 mg |
| I. Aspartame | 1.00 mg |
| L. Fruits Flavor | 3.00 ng |
| M. Glycine | 22.00 mg |
| Total weight of effervescent tablet | 1000.00 mg |

### EXAMPLE 3A

Effervescent tablets of 400 mg SAMe ion/tablet

Composition based on SAMe sulphate p-toluene sulphonate

| | |
|---|---|
| A. SAMe sulphate p-toluene sulphonate | 800.00 mg |
| B. Calcium oxide not present | 00.00 mg |
| C. Anhydrous Calcium chloride | 40.00 mg |
| D. Sodium bicarbonate | 70.00 mg |
| E. Anhydrous Tartaric acid | 45.00 mg |
| F. Anhydrous Malic acid | 35.00 mg |
| G. Sorbitol | 265.00 mg |
| H. Mannitol | 159.00 mg |
| I. Aspartame | 1.00 mg |
| L. Fruits Flavor | 3.00 ng |
| M. Glycine | 22.00 mg |
| Total weight of effervescent tablet | 1000.00 mg |

The quantities relate to the preparation of a standard industrial batch of 300.00 kg of tablets.

The tablets were prepared as described in Example 1 using the components and quantities indicated above.

Stability tests on effervescent tablets were performed at 60°C for one months and for a single batch. The samples were stored in alu/alu blister.

**Table 5**

| Batch 003 - effervescent tablets of 400 mg SAMe ion/tablet (qualitative/quantitative composition in Example 3) | | | | | |
|---|---|---|---|---|---|
| Batch (T/t)¹ | Moisture content % (K. Fischer) | S,S % | AD² (%) | MTAD³ (%) | SAMe⁴ |
| 003 (20/0) | 0.65 | 79.98 | 0.21 | 0.35 | 407.23 |
| 003A (60/1) | 0.59 | 64.30 | 2.98 | 5.34 | 400.01 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

The data in Table 3 show that the tablets have optimum stability.

**Table 6**

| Batch 003A - effervescent tablets of 400 mg SAMe ion/tablet (qualitative/quantitative composition in Example 3A) | | | | | |
|---|---|---|---|---|---|
| Batch (T/t)¹ | Moisture content % (K.Fischer) | S,S % | AD² (%) | MTAD³ (%) | SAMe⁴ |
| 003A (20/0) | 0.67 | 79.56 | 0.29 | 0.56 | 407.23 |
| 003AA (60/1) | 0.87 | 49.89 | 13.58 | 21.99 | 298.78 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

The data in Table 3 show that the tablets have not good stability

### EXAMPLE 4

Mouth-soluble granulates of 400 mg SAMe ion/bags

Composition based on SAMe sulphate p-toluene sulphonate

| | |
|---|---|
| A. SAMe sulphate p-toluene sulphonate | 802.20 mg |
| B. Calcium oxide | 39.60 mg |
| C. Anhydrous Calcium chloride | 39.60 mg |
| D. Potassium carbonate | 140.00 mg |
| E. Anhydrous Citric acid | 74.80 mg |
| F. Anhydrous Adipic acid | 84.58 mg |
| G. Dextrose | 484.77 mg |
| H. Anhydrous granular Sorbitol | 283.19 mg |
| I. Sucralose | 1.76 mg |
| L. Lemon Flavor | 5.50 ng |
| M. Benzoic acid | 44.00 mg |
| Total weight of bags | 2000.00 mg |

The quantities relate to the preparation of a standard industrial batch of 300.00 kg of bags.

The tablets were prepared in the manner described in Example 2 using the components and quantities indicated above.

Stability tests on effervescent tablets were performed at only 60°C for one months and for a single batch. The samples were stored in alu/alu bags.

**Table 7**

| Batch 004 - mouth-soluble granulates of 400 mg SAMe ion/bags (qualitative/quantitative composition in Example 4) | | | | | |
|---|---|---|---|---|---|
| Batch (T/t)¹ | Moisture content % (K. Fischer) | S,S % | AD² (%) | MTAD³ (%) | SAMe⁴ |
| 004 (20/0) | 0.74 | 83.5 | 0.29 | 0.13 | 404.34 |
| 004A (60/1) | 0.67 | 68.45 | 2.67 | 5.87 | 397.74 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

The data in Table 4 show that the tablets have optimum stability

### EXAMPLE 5

Effervescent tablets of 200 mg SAMe ion/tablet

Composition based on SAMe sulphate p-toluene sulphonate

| | |
|---|---|
| A. SAMe sulphate p-toluene sulphonate | 400.00 mg |
| B. DL Phosphoserine | 25.00 mg |
| C. Calcium oxide | 20.00 mg |
| D. Anhydrous Calcium chloride | 20.00 mg |
| E. Potassium bicarbonate | 75.00 mg |
| F. Anhydrous Adipic acid | 40.00 mg |
| G. Anhydrous Malic acid | 30.00 mg |
| H. Sorbitol | 270.00 mg |
| I. Xylitol | 144.00 mg |
| L. Sucralose | 1.00 mg |
| M. Lemon Flavor | 3.00 ng |
| N. Sodium fumarate | 22.00 mg |
| Total weight of effervescent tablet | 1050.00 mg |

The quantities relate to the preparation of a standard industrial batch of 300.00 kg of tablets.

The tablets were prepared in the manner described in Example 1 using the components and quantities indicated above.

Stability tests on effervescent tablets were performed at only 60°C for one months and for a single batch. The samples were stored in alu/alu blister.

**Table 8**

| Batch 005 - Effervescent tablets of 200 mg/ion/tablet (qualitative/quantitative composition in Example 5) | | | | | | |
|---|---|---|---|---|---|---|
| Batch (T/t)¹ | Moisture content % (K. Fischer) | S,S % | AD² (%) | MTAD³ (%) | SAMe⁴ | DL-phosphoserine |
| 005 (20/0) | 0.72 | 81.6 | 0.29 | 0.24 | 211.32 | 25.54 |
| 005A (60/1) | 0.49 | 75.83 | 3.62 | 4.91 | 200.14 | 24.83 |

| | | | | | | |
|---|---|---|---|---|---|---|
| ¹ Temperature (°C)/time (months); ² adenosine; ³ methylthioadenosine; ⁴ SAMe sulphate p-toluene sulphonate (mg/tablet); | | | | | | |

The data in Table 5 show that the tablets have optimum stability.

### EXAMPLE 6

Mouth-soluble granulates of 200 mg SAMe ion/bags

Composition based on SAMe sulphate p-toluene sulphonate

| | |
|---|---|
| A. SAMe sulphate p-toluene sulphonate | 401.10 mg |
| B. N-acetyl-L-glutamine | 25.00 mg |
| C. Phosphatidylserine | 50.00 mg |
| D. Calcium oxide | 19.80 mg |
| E. Anhydrous Calcium chloride | 19.80 mg |
| F. Sodium carbonate | 74.10 mg |
| G. Anhydrous Citric acid | 37.40 mg |
| H. Anhydrous Adipic acid | 37.40 mg |
| I. Lactose | 264.77 mg |
| L. Anhydrous granular Sorbitol | 220.00 mg |
| M. Sucralose | 0.88 mg |
| N. Lemon Flavor | 2.75 ng |
| O. Sodium fumarate | 22.00 mg |
| Total weight of bags | 1175.00 mg |

The quantities relate to the preparation of a standard industrial batch of 300.00 kg of bags.

The bags were prepared in the manner described in Example 2 using the components and quantities indicated above.

Stability tests on effervescent tablets were performed at only 60°C for one month and for a single batch. The samples were stored in alu/alu bags.

**Table 9**

| Batch 006 - Mouth-soluble granulates of 200 mg SAMe ion/bags (qualitative/quantitative composition in Example 6) | | | | | | | |
|---|---|---|---|---|---|---|---|
| Batch (T/t)¹ | Moisture content % (K. Fischer) | S,S % | AD² (%) | MTA D³ (%) | SAMe ⁴ | N-acetyl-L-glutamine mg | Phosphatidylserine mg |
| 006 (20/0) | 0.59 | 81.11 | 0.37 | 0.22 | 206.89 | 26.23 | 53.23 |
| 006A (60/1) | 0.53 | 72.4 | 0.49 | 0.59 | 199.73 | 25.94 | 52.24 |

| | | | | | | | |
|---|---|---|---|---|---|---|---|
| ¹ Temperature (°C)/time (months); ² adenosine; ³ methylthioadenosine; ⁴ SAMe sulphate p-toluene sulphonate (mg/bags); | | | | | | | |

The data in Table 6 indicate that the tablets have optimum stability.

The quantities relate to the preparation of a standard industrial batch of 300.00 kg of tablets

### EXPERIMENTAL PART

### Stability tests on the finished product

Stability at 40°C 75% RH (STRESS TEST) and at ambient temperature over a long period (SHELF LIFE) for the compositions of Examples 1, 2, 3, 4, 5, 6 were evaluated for changes in appearance (essentially change in colour), titre of SAMe sulphate p-toluene sulphonate and other active ingredients (mg/tablet; mg/bags), increase in degradation purities, moisture content (K.F.) and % of the active (SS)-(+)-S-adenosyl-L-methionine diastereoisomer; the presence of any degradation products, which can be substantially identified as adenosine and methylthioadenosine, expressed as a percentage with respect to the weight of SAMe-toluene sulphonate per tablet, was further checked by HPLC.

### STRESS TEST

The tablets were prepared in stoppered glass bottles and enclosed in such a way as to reproduce the conditions of final packaging (generally aluminium/aluminium blister).

The samples so prepared were stored for six months in a stove thermostated to a temperature of 40 ± 2°C and 75% RH.

Three samples from one batch were used for the 200/400 mg effervescent tablets (Examples 1, 3, 5) and three samples from one batch were used for the mouth-soluble granulates (Examples 2, 4, 6) of 200/400 mg SAMe ion/bags.

The following tables (10 - 18) report the results of the stress test.

**Table 10**

| Batch 001 - Effervescent tablets of 200 mg SAMe ion/tablet (Example 1) | | | | | |
|---|---|---|---|---|---|
| Batch (T/t)¹ | Moisture content % (K.Fischer) | S,S % | AD² (%) | MTAD³ (%) | SAMe⁴ mg |
| 001 (20/0) | 0.54 | 82.2 | 0.32 | 0.45 | 202.23 |
| 001A (40/1) | 0.65 | 75.4 | 0.36 | 0.65 | 201.76 |
| 001B (40/3) | 0.53 | 73.8 | 0.79 | 0.74 | 200.43 |
| 001C (40/6) | 0.60 | 68.0 | 0.87 | 1.21 | 197.54 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

**Table 11**

| Batch 002 - Mouth-soluble granulates of 200 mg SAMe ion/bags (Example 2) | | | | | |
|---|---|---|---|---|---|
| Batch (T/t)¹ | Moisture content % (K. Fischer) | S,S % | AD² (%) | MTAD³ (%) | SAMe⁴ mg |
| 002 (20/0) | 0.57 | 80.1 | 0.22 | 0.32 | 204.54 |
| 002A (40/1) | 0.62 | 76.4 | 0.54 | 0.76 | 203.65 |
| 002B (40/3) | 0.77 | 73.8 | 0.59 | 0.82 | 202.61 |
| 002C (40/6) | 0.79 | 69.6 | 0.87 | 0.99 | 198.01 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

**Table 12**

| Batch 003 - Effervescent tablets of 400 mg SAMe ion/tablet (Example 3) | | | | | |
|---|---|---|---|---|---|
| Batch (T/t)¹ | Moisture content % (K. Fischer) | S,S % | AD² (%) | MTAD³ (%) | SAMe⁴ mg |
| 003 (20/0) | 0.65 | 79.9 | 0.21 | 0.35 | 407.23 |
| 003A (40/1) | 0.73 | 75.8 | 0.57 | 0.49 | 405.98 |
| 003B (40/3) | 0.69 | 73.6 | 0.79 | 0.79 | 404.88 |
| 003C (40/6) | 0.76 | 67.5 | 1.08 | 0.98 | 401.81 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

**Table 13**

| Batch 004 - Mouth-soluble granulates of 400 mg SAMe ion/bags (Example 4) | | | | | |
|---|---|---|---|---|---|
| Batch (T/t)¹ | Moisture content % (K. Fischer) | S,S % | AD² (%) | MTAD³ (%) | SAMe⁴ mg |
| 004 (20/0) | 0.74 | 83.5 | 0.29 | 0.13 | 404.34 |
| 004A (40/1) | 0.45 | 75.9 | 0.59 | 0.35 | 402.76 |
| 004B (40/3) 004C (40/6) | 0.49 | 71.7 | 0.58 | 0.57 | 400.61 |
| | 0.76 | 69.9 | 0.99 | 0.78 | 398.94 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

**Table 14**

| Batch 05 - Effervescent tablets of 200 mg SAMe ion/tablet (Example 5) | | | | | | |
|---|---|---|---|---|---|---|
| Batch (T/t)¹ | Moisture content % (K. Fischer) | S,S % | AD² (%) | MTAD³ (%) | SAMe ⁴ mg | DL-phosphoserine Mg |
| 005 (20/0) | 0.72 | 81.6 | 0.29 | 0.24 | 211.32 | 25.54 |
| 005A (40/1) | 0.59 | 73.3 | 0.59 | 0.54 | 209.75 | 24.65 |
| 005B (40/3) | 0.61 | 70.8 | 0.69 | 0.78 | 207.56 | 24.55 |
| 005C (40/6) | 0.87 | 68.9 | 0.77 | 1.65 | 205.65 | 24.32 |

| | | | | | | |
|---|---|---|---|---|---|---|
| ¹ Temperature (°C)/time (months); ² adenosine; ³ methylthioadenosine; ⁴ SAMe sulphate p-toluene sulphonate (mg/tablet); | | | | | | |

**Table 15**

| Batch 06 - Mouth-soluble granulates of 200 mg SAMe ion/bags (Example 6) | | | | | | | |
|---|---|---|---|---|---|---|---|
| ot (T/t)¹ | Moisture content % (K. Fischer) | S,S % | AD² (%) | MTAD ³ (%) | SAMe⁴ mg | N-acetyl-L-glutamine mg | Phosphatidylserine mg |
| 006 (20/0) | 0.59 | 81.1 | 0.37 | 0.22 | 206.89 | 26.23 | 53.23 |
| 006A (40/1) | 0.55 | 73.8 | 0.35 | 0.59 | 205.89 | 25.02 | 52.02 |
| 006B (40/3) | 0.65 | 70.1 | 0.64 | 0.87 | 203.65 | 24.98 | 51.98 |
| 006C (40/6) | 074 | 68.3 | 0.95 | 1.17 | 200.65 | 24.95 | 51.45 |

| | | | | | | | |
|---|---|---|---|---|---|---|---|
| ¹ Temperature (°C)/time (months); ² adenosine; ³ methylthioadenosine; ⁴ SAMe sulphate p-toluene sulphonate (mg/bags); | | | | | | | |

From the stability data at 40°C and 75% RH (STRESS TEST) it is evident that all the batches examined after six months suffered degradation equal to approximately 7.0% of both SAMe and the other active ingredients with a reduction of approximately 13% in the active (SS)-(+)-S-adenosyl-L-methionine diastereoisomer.

**Table 16**

| Batch 001A - Effervescent tablets of 200 mg SAMe ion/tablet (Example 1A) | | | | | |
|---|---|---|---|---|---|
| Batch (T/t)¹ | Moisture content % (K. Fischer) | S,S % | AD² (%) | MTAD³ (%) | SAMe⁴ mg |
| 001A (20/0) | 0.76 | 81.7 | 0.32 | 0.76 | 202.23 |
| 001AA (40/1) | 0.87 | 55.4 | 2.36 | 8.89 | 178.98 |
| 001AB (40/3) | 0.93 | 51.8 | 7.65 | 15.84 | 154.63 |
| 001AC (40/6) | 1.60 | 49.7 | 13.65 | 21.21 | 121.74 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

**Table 17**

| Batch 002A - Mouth-soluble granulates of 200 mg SAMe ion/bags (Example 2A) | | | | | |
|---|---|---|---|---|---|
| Batch (T/t)¹ | Moisture content % (K. Fischer) | S,S % | AD² (%) | MTAD³ (%) | SAMe⁴ mg |
| 002A (20/0) | 0.55 | 82.4 | 0.29 | 0.43 | 203.74 |
| 002AA (40/1) | 0.68 | 58.4 | 3.84 | 7.83 | 168.05 |
| 002AB (40/3) | 0.97 | 53.7 | 8.49 | 17.44 | 147.72 |
| 002AC (40/6) | 1.65 | 49.9 | 14.93 | 22.79 | 119.61 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

**Table 18**

| Batch 003A - Effervescent tablets of 400 mg SAMe ion/tablet (Example 3A) | | | | | |
|---|---|---|---|---|---|
| Batch (T/t)¹ | Moisture content % (K. Fischer) | S,S % | AD² (%) | MTAD³ (%) | SAMe⁴ mg |
| 003 (20/0) | 0.67 | 79.5 | 0.29 | 0.56 | 407.23 |
| 003AA (40/1) | 0.93 | 57.5 | 3.05 | 6.59 | 345.67 |
| 003AB (40/3) | 1.49 | 53.6 | 7.69 | 15.20 | 304.85 |
| 003AC (40/6) | 1.78 | 48.5 | 12.88 | 20.67 | 267.89 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

From the stability data at 40°C and 75% RH (STRESS TEST) it is evident that all the batches without calcium oxide after six months suffered degradation equal to approximately 40% of both SAMe and the other active ingredients with a reduction of approximately 30% in the active (SS)-(+)-S-adenosyl-L-methionine diastereoisomer.

### SHELF LIFE

The tablets were packed in stoppered glass bottles and enclosed so as to reproduce the conditions of final packaging (generally aluminium/aluminium blister).

The samples were selected in the same way and in the same quantities as described for the stress test and kept in an environment thermostated to a temperature of 25 ± 2°C and a humidity of 60% RH.

Three samples from one batches were used for the 200/400 mg effervescent tablets (Examples 1, 3, 5) and three samples from one batch were used for the mouth-soluble granulates (Examples 2, 4, 6) of 200/400 mg SAMe ion/bags.

The following tables 19 - 27 show the results for SHELF LIFE.

**Table 19**

| Batch 001 - Effervescent tablets of 200 mg SAMe ion/tablet (Example 1) | | | | | |
|---|---|---|---|---|---|
| Batch (T/t)¹ | Moisture content % (K. Fischer) | S,S % | AD² (%) | MTAD³ (%) | SAMe⁴ mg |
| 001 (20/0) | 0.54 | 82.2 | 0.32 | 0.45 | 202.23 |
| 001A (25/3) | 0.76 | 77.3 | 0.49 | 0.37 | 202.00 |
| 001B (25/6) | 0.58 | 74.5 | 0.57 | 0.55 | 201.89 |
| 001C (25/12) | 0.66 | 72.9 | 0.63 | 0.68 | 200.76 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

**Table 20**

| Batch 002 - Mouth-soluble granulates of 200 mg SAMe ion/bags (Example 2) | | | | | |
|---|---|---|---|---|---|
| Batch (T/t)¹ | Moisture content % (K. Fischer) | S,S % | AD² (%) | MTAD³ (%) | SAMe⁴ mg |
| 002 (20/0) | 0.57 | 80.1 | 0.22 | 0.32 | 204.54 |
| 002A (25/3) | 0.66 | 76.6 | 0.36 | 0.57 | 203.78 |
| 002B (25/6) | 0.59 | 74.5 | 0.54 | 0.65 | 203.00 |
| 002C (25/12) | 0.36 | 73.4 | 0.67 | 0.76 | 202.54 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

**Table 21**

| Batch 003 - Effervescent tablets of 400 mg SAMe ion/tablet (Example 3) | | | | | |
|---|---|---|---|---|---|
| Batch (T/t)¹ | Moisture content % (K. Fischer) | S,S % | AD² (%) | MTAD³ (%) | SAMe⁴ mg |
| 003 (20/0) | 0.65 | 79.9 | 0.21 | 0.35 | 407.23 |
| 003A (25/1) | 0.45 | 74.5 | 0.35 | 0.55 | 407.44 |
| 003B (25/6) | 0.59 | 73.6 | 0.63 | 0.59 | 406.32 |
| 003C (25/12) | 0.65 | 72.65 | 0.76 | 0.78 | 405.53 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

**Table 22**

| Batch 004 - Mouth-soluble granulates of 400 mg SAMe ion/bags (Example 4) | | | | | |
|---|---|---|---|---|---|
| Batch (T/t)¹ | Moisture content % (K. Fischer) | S,S % | AD² (%) | MTAD³ (%) | SAMe⁴ mg |
| 004 (20/0) | 0.70 | 80.0 | 0.41 | 0.20 | 410.24 |
| 004A (25/3) | 0.64 | 75.7 | 0.54 | 0.47 | 408.65 |
| 004B (25/6) | 0.55 | 73.7 | 0.69 | 0.58 | 407.56 |
| 004C (25/12) | 0.43 | 74.4 | 0.83 | 0.85 | 406.58 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

**Table 23**

| Batch 05 - Effervescent tablets of 200 mg SAMe ion/tablet (Example 5) | | | | | | |
|---|---|---|---|---|---|---|
| Batch (T/t)¹ | Moisture content % (K. Fischer) | S,S % | AD² (%) | MTAD³ (%) | SAMe⁴ mg | DL-phosphoserine mg |
| 005 (20/0) | 0.61 | 80.0 | 0.52 | 0.53 | 410.54 | 2.03 |
| 005A (25/3) | 0.57 | 75.4 | 0.55 | 0.58 | 409.65 | 2.03 |
| 005B (25/6) | 0.51 | 74.3 | 0.67 | 0.69 | 408.56 | 2.00 |
| 005C (25/12) | 0.48 | 73.0 | 0.76 | 0.78 | 407.98 | 1.95 |

| | | | | | | |
|---|---|---|---|---|---|---|
| ¹ Temperature (°C)/time (months); ² adenosine; ³ methylthioadenosine; ⁴ SAMe sulphate p-toluene sulphonate (mg/tablet); | | | | | | |

**Table 24**

| Batch 06 - Mouth-soluble granulates of 200 mg SAMe ion/bags (Example 6) | | | | | | | |
|---|---|---|---|---|---|---|---|
| ot (T/t)¹ | Moisture content % (K. Fischer) | S,S % | AD² (%) | MTAD³ (%) | SAMe⁴ mg | N-acetyl-L-glutamine mg | Phosphatidylserine mg |
| 006 (20/0) | 0.59 | 81.1 | 0.37 | 0.22 | 206.89 | 26.23 | 53.23 |
| 006A (25/3) | 0.65 | 74.6 | 0.54 | 0.47 | 204.76 | 25.02 | 52.02 |
| 006B (25/6) | 0.78 | 73.8 | 0.49 | 0.68 | 204.33 | 24.92 | 51.87 |
| 006C (25/12) | 0.63 | 72.6 | 0.68 | 0.75 | 203.77 | 24.65 | 51.66 |

| | | | | | | | |
|---|---|---|---|---|---|---|---|
| ¹ Temperature (°C)/time (months); ² adenosine; ³ methylthioadenosine; ⁴ SAMe sulphate p-toluene sulphonate (mg/bags); | | | | | | | |

From the stability data at 25°C and 60% RH (SHELF LIFE) it is evident that all the batches examined after twelve months suffered very little degradation of the SAMe with a reduction of approximately 10% in the active (SS)-(+)-S-adenosyl-L-methionine diastereoisomer.

**Table 25**

| 001A - Effervescent tablets of 200 mg SAMe ion/tablet (Example 1A) | | | | | |
|---|---|---|---|---|---|
| Batch (T/t)¹ | Moisture content % (K. Fischer) | S,S % | AD² (%) | MTAD³ (%) | SAMe⁴ mg |
| 001A (20/0) | 0.76 | 81.7 | 0.32 | 0.76 | 202.23 |
| 001AA (25/3) | 0.78 | 67.3 | 1.49 | 2.03 | 189.34 |
| 001AB (25/6) | 1.68 | 54.2 | 5.48 | 7.99 | 164.99 |
| 001AC (25/12) | 1.86 | 50.9 | 10.63 | 13.49 | 148.48 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

**Table 26**

| Batch 002A - Mouth-soluble granulates of 200 mg SAMe ion/bags (Example 2A) | | | | | |
|---|---|---|---|---|---|
| Batch (T/t)¹ | Moisture content % (K. Fischer) | S,S % | AD² (%) | MTAD³ (%) | SAMe⁴ mg |
| 002A (20/0) | 0.55 | 82.4 | 0.29 | 0.43 | 203.74 |
| 002AA (25/3) | 0.73 | 68.2 | 1.39 | 2.34 | 187.31 |
| 002AB (25/6) | 1.48 | 56.8 | 5.00 | 7.76 | 168.59 |
| 002AC (25/12) | 1.56 | 51.3 | 10.01 | 12.39 | 150.46 |

| | | | | | |
|---|---|---|---|---|---|
| ¹ Temperature (°C)/time (months); ² adenosine; ³ methylthioadenosine; ⁴ SAMe sulphate p-toluene sulphonate (mg/bags); | | | | | |

**Table 27**

| Batch 003A - Effervescent tablets of 400 mg SAMe ion/tablet (Example 3A) | | | | | |
|---|---|---|---|---|---|
| Batch (T/t)¹ | Moisture content % (K. Fischer) | S,S % | AD² (%) | MTAD³ (%) | SAMe⁴ mg |
| 003A (20/0) | 0.67 | 79.5 | 0.29 | 0.56 | 407.23 |
| 003AA (25/1) | 0.75 | 64.5 | 2.35 | 2.33 | 389.73 |
| 003AB (25/6) | 0.79 | 53.9 | 6.62 | 8.29 | 356.89 |
| 003AC (25/12) | 1.25 | 49.29 | 11.22 | 15.79 | 308.49 |

| | | | | | |
|---|---|---|---|---|---|
| ¹ Temperature (°C)/time (months); ² adenosine; ³ methylthioadenosine; ⁴ SAMe sulphate p-toluene sulphonate (mg/tablet); | | | | | |

From the stability data at 25°C and 60% RH (SHELF LIFE) it is evident that all the batches without calcium oxide after twelve months suffered very hard degradation of the SAMe with a reduction of approximately 30% in the active (SS)-(+)-S-adenosyl-L-methionine diastereoisomer.

## Claims

1. A pharmaceutical or nutraceutical composition in form of effervescent tablets or mouth-soluble granulates comprising SAMe, an acid, a carbon dioxide source, calcium oxide and optionally pharmaceutically acceptable excipients.

2. A composition according to Claim 1, in which SAMe is S-adenosyl methionine para-toluene sulphonate, S-adenosyl methionine-1,4-butene disulphonate, S-adenosyl methionine sulphate, S-adenosyl methionine tosylate, S-adenosyl methionine phytate.

3. A composition according to claim 1 or 2, in which SAMe is present in a quantity from approximately 10 to approximately 95% by weight of the composition.

4. A composition according to claim 3, in which SAMe is present in a quantity approximately from 30 to 90% by weight of the composition.

5. A composition according to any one of claims from 1 to 4 wherein the acid is selected from citric, tartaric, malic, maleic, fumaric or adipic acid.

6. A composition according to any one of claims 1 to 5 wherein the carbon dioxide source is selected from an alkali or alkaline-earth metal carbonate or bicarbonate or sodium glycine carbonate or mixtures thereof.

7. A composition according to any one of claims 1 to 6, in which the mixture of calcium oxide/bicarbonate and/or carbonate is present in a quantity from approximately 1 to approximately 50% by weight of the composition.

8. A composition according to claim 7, in which the mixture of calcium oxide/bicarbonate and/or carbonate is present in a quantity from approximately 5 to approximately 30% by weight of the composition.

9. A composition according to any one of claims 1 to 8, comprising at least one further active ingredient selected from phosphatidylserine, DL-phosphorylserine, N-acetyl-L-glutamine and L - Carnitine.

10. A composition according to any one of claims 1 to 9 comprising glycine and/or leucine and/or PEG 200 - 8000 as excipients.

11. A process for the preparation of a tablet of claims 1-10 comprising the following steps:
a) mixing of the SAMe and/or their salts with a mixture of calcium oxide/bicarbonate and/or carbonate and pharmaceutically acceptable excipients;
b) precompression and subsequent granulation of the mixture from a);
c) mixing of the granulate from b) with other excipients;
d) compression of the mixture from c), with the optional addition of sweeteners and/or flavours.

12. A process according to Claim 11, in which the absolute moisture content is lower than 40%-50% and the temperature is maintained at 20°C -25°C.
